# EUROPEAN PATENT APPLICATION

(11) **EP 4 790 030 A1**
(43) Date of publication of application: **12.08.2026**
(21) Application number: 25759265.9
(22) Date of filing: 05.03.2025
(51) Int. Cl.: C03B 5/235, C03B 5/00, C01B 13/02, C25B 1/04, C25B 9/00, C25B 15/08, C25B 9/65, C01B 3/38, B01D 45/16, B01D 53/18, F27D 17/15, H02J 7/35, C07C 1/12

(54) **ZERO-CARBON GLASS KILN PROCESS**

(30) Priority: 22.11.2024 CN 202411679279
(71) Applicant: Shanghai Yuanhan Energy & Chemical Technology Co., Ltd., Shanghai 200135 (CN)
(72) Inventor: ZHANG, Yunfeng, Pudong New Area , Shanghai 200135 (CN); ZHANG, Xiangquan, Pudong New Area , Shanghai 200135 (CN); WU, Wenjun, Pudong New Area , Shanghai 200135 (CN)
(74) Representative: Dai, Simin
(86) International application number: PCT/CN2025/080794
(87) International publication number: WO 2026/108023

(57) **Abstract**

Provided is a zero-carbon glass furnace process, a system required for the process includes a photovoltaic power generation unit, an air separation unit, a water electrolysis hydrogen production unit, a mixer, a methanation unit, a first waste heat boiler, a reforming unit, a glass furnace, a second waste heat boiler, and a dedusting and desulfurization unit. The present disclosure adopts green electricity to produce green oxygen, green nitrogen and green hydrogen; flue gas of the glass furnace is circularly enriched into a high concentration of carbon dioxide (95.0v% or higher), which is methanized with green hydrogen to produce methane, and the methane is reformed with carbon dioxide and water vapor to produce carbon monoxide and hydrogen; the carbon monoxide and hydrogen are used as fuel of the glass furnace, the oxygen and carbon dioxide are mixed into carbon-based enriched oxygen as a combustion aid of the glass furnace, and the excess carbon dioxide is sold externally, achieving the purpose of green, energy-saving, almost nitrogen oxide-free and zero carbon.

## Description

### TECHNICAL FIELD

The present disclosure relates to the technical field of glass furnace, and in particular, to a zero-carbon glass furnace process.

### BACKGROUND

Greenhouse gas emissions are the most important factor causing global warming. The greenhouse effect caused by carbon dioxide accounts for more than 70v% of that from all greenhouse gases, so carbon dioxide emission reduction is an urgent problem to be solved, which is important for controlling the greenhouse effect and slowing down global warming.

Currently, flue gas from glass furnace is basically discharged directly into atmosphere. Although carbon emission share of glass industry is not large, it is a high-energy-consumption and high-emission industry, and CO₂ emissions are not optimistic.

### SUMMARY

The purpose of the present invention is to provide a zero-carbon glass furnace process to address the deficiencies of the prior art.

To achieve the above purpose, the present disclosure adopts the following technical scheme:

A zero-carbon glass furnace process is provided. A system required for the process includes a photovoltaic power generation unit, an air separation unit, a water electrolysis hydrogen production unit, a mixer, a methanation unit, a first waste heat boiler, a reforming unit, a glass furnace, a second waste heat boiler, and a dedusting and desulfurization unit.

The photovoltaic power generation unit is configured to produce green electricity to supply electricity to the air separation unit and the water electrolysis hydrogen production unit.

The air separation unit is configured to produce oxygen and nitrogen.

The water electrolysis hydrogen production unit is configured to produce hydrogen and oxygen.

The mixer is configured to mix the oxygen from the air separation unit, the oxygen from the water electrolysis hydrogen production unit, and circulating flue gas from the glass furnace into carbon-based enriched oxygen.

The methanation unit is configured to methanize the hydrogen from the water electrolysis hydrogen production unit and circulating flue gas from the dedusting and desulfurization unit.

The first waste heat boiler is configured to recover waste heat of methane containing saturated water vapor from the methanation unit and by-produce steam.

The reforming unit is configured to subject the methane containing saturated water vapor from the first waste heat boiler and a portion of the circulating flue gas from the glass furnace to a methane reforming reaction.

The glass furnace is configured to produce molten glass using carbon monoxide and hydrogen from the reforming unit as fuel and the carbon-based enriched oxygen from the mixer as a combustion aid.

The second waste heat boiler is configured to recover waste heat of the circulating flue gas from the glass furnace and by-produce steam.

The dedusting and desulfurization unit is configured to dedust and desulfurize circulating flue gas from the second waste heat boiler.

The photovoltaic power generation unit is connected to the air separation unit and the water electrolysis hydrogen production unit, respectively; a nitrogen outlet of the air separation unit is connected to a tin bath of the glass furnace and to a nitrogen product storage tank, respectively, and a pressure reducing valve is provided on a connecting pipe between the nitrogen outlet of the air separation unit and the tin bath of the glass furnace; an oxygen outlet of the air separation unit and an oxygen outlet of the water electrolysis hydrogen production unit are both connected to the mixer, a pressure reducing valve is provided on a connecting pipe between the oxygen outlet of the water electrolysis hydrogen production unit and the mixer, a connecting pipe between the oxygen outlet of the air separation unit, the oxygen outlet of the water electrolysis hydrogen production unit, and the mixer is provided with a flow detector, a temperature detector, a pressure detector, an oxygen purity detector, and a flow regulating valve; a hydrogen outlet of the water electrolysis hydrogen production unit is connected to the methanation unit; a carbon-based enriched oxygen outlet of the mixer is switchably connected to a heat storage grid A/B of the glass furnace and then connected to the glass furnace; a methane outlet of the methanation unit is connected to the first waste heat boiler; a methane outlet of the first waste heat boiler is switchably connected to a heat storage grid A/B of the reforming unit and then connected to the reforming unit, a pressure reducing valve is provided on a connecting pipe between the methane outlet of the first waste heat boiler and the heat storage grid A/B of the reforming unit, and a steam outlet of the first waste heat boiler is connected to a steam utilizer; a carbon monoxide and hydrogen outlet of the reforming unit is connected to the glass furnace; one of circulating flue gas outlets of the glass furnace is switchably connected to the heat storage grid B/A of the glass furnace and then connected to the second waste heat boiler and the mixer, one of the circulating flue gas outlets of the glass furnace is switchably connected to the heat storage grid A/B of the reforming unit and then connected to the reforming unit, and one of the circulating flue gas outlets of the glass furnace is switchably connected to a heat storage grid B/A of the reforming unit and then connected to the dedusting and desulfurization unit, respectively, a pressure booster is provided on a connecting pipe between the heat storage grid B/A of the glass furnace, the second waste heat boiler, and the mixer, a connecting pipe between the heat storage grid B/A of the glass furnace and the mixer is provided with a flow detector, a temperature detector, a pressure detector, a carbon dioxide purity detector, and a flow regulating valve, a pressure booster is provided on the connecting pipe between the glass furnace, the heat storage grid A/B of the reforming unit, and the heat storage grid B/A of the reforming unit, and a pressure reducing valve is provided on a connecting pipe between the heat storage grid B/A of the reforming unit and the dedusting and desulfurization unit; a circulating flue gas outlet of the second waste heat boiler is connected to the dedusting and desulfurization unit; circulating flue gas outlets of the dedusting and desulfurization unit are connected to the methanation unit and a carbon dioxide product storage tank, respectively, and a pressure booster is provided on a connecting pipe between one of the circulating flue gas outlets of the dedusting and desulfurization unit and the methanation unit.

The process comprises operations of:
(1) the photovoltaic power generation unit producing the green electricity to supply the electricity for the air separation unit and the water electrolysis hydrogen production unit; the air separation unit producing oxygen and nitrogen with the green electricity supplied by the photovoltaic power generation unit as the electricity, wherein the oxygen is fed into the mixer, a portion of the nitrogen is depressurized and then fed into the tin bath of the glass furnace as protective gas, and the rest is sold outside; the water electrolysis hydrogen production unit producing hydrogen and oxygen with the green electricity supplied by the photovoltaic power generation unit as the electricity, wherein the hydrogen is fed into the methanation unit, and the oxygen is fed into the mixer after depressurization;
(2) the mixer mixing the oxygen from the air separation unit, the oxygen from the water electrolysis hydrogen production unit, and the circulating flue gas from the glass furnace into the carbon-based enriched oxygen, and using the carbon-based enriched oxygen as the combustion aid for the glass furnace;
(3) the methanation unit methanizing the hydrogen from the water electrolysis hydrogen production unit and the circulating flue gas from the dedusting and desulfurization unit to produce the methane containing saturated water vapor, and feeding the methane containing saturated water vapor into the first waste heat boiler;
(4) the first waste heat boiler recovering the waste heat of the methane containing saturated water vapor from the methanation unit and by-producing the steam; selling the steam externally, and feeding depressurized methane containing saturated water vapor after waste heat recovery to the reforming unit;
(5) the reforming unit subjecting the methane containing saturated water vapor from the first waste heat boiler and the portion of the circulating flue gas from the glass furnace to the methane reforming reaction; the circulating flue gas from the glass furnace entering into the reforming unit in two ways, one way entering into a heat storage grid of one side of the reforming unit, providing heat for the heat storage grid of the one side of the reforming unit to provide heat for raw gas of the reforming unit in a next round, and after heat exchange, the circulating flue gas being depressurized and fed into the dedusting and desulfurization unit; the other way entering into a heat storage grid on the other side of the reforming unit along with the methane containing saturated water vapor from the first waste heat boiler, the heat storage grid on the other side of the reforming unit being already heated by the circulating flue gas of a previous round, two sides of the heat storage grids working in rotation, and the raw gas fed into the reforming unit being heated by heat of the heat storage grid on the other side of the reforming unit, in a reaction bed of the reforming unit, under a certain pressure and a certain catalyst, methane, carbon dioxide, and water vapor undergoing the methane reforming reaction to produce the carbon monoxide and the hydrogen, and feeding the carbon monoxide and the hydrogen into the glass furnace as the fuel;
(6) the glass furnace producing the molten glass using the carbon monoxide and the hydrogen from the reforming unit as the fuel and the carbon-based enriched oxygen from the mixer as the combustion aid; the carbon-based enriched oxygen from the mixer first passing through a heat storage grid on one side of the glass furnace, the heat storage grid on the one side of the glass furnace being already heated by the previous round of the circulating flue gas, two sides of the heat storage grids working in rotation, and the carbon-based enriched oxygen from the mixer being heated to a certain temperature and then fed into the glass furnace;
   after the flue gas of the glass furnace is circularly enriched for a certain period of time, and a carbon dioxide concentration reaching an equilibrium; the circulating flue gas being divided into two ways, one way passing through a heat storage grid on the other side of the glass furnace, providing heat for the heat storage grid on the other side of the glass furnace to provide heat for the carbon-based enriched oxygen from the mixer in the next round, and after heat exchange, the circulating flue gas of this way being pressurized and fed into the mixer and the second waste heat boiler; the other way not passing through the heat storage grid of the glass furnace, and being pressurized and fed into the reforming unit;
(7) the second waste heat boiler recovering the waste heat of the circulating flue gas from the glass furnace and by-producing the steam; selling the steam externally, and feeding the circulating flue gas after waste heat recovery to the dedusting and desulfurization unit; and
(8) the dedusting and desulfurization unit dedusting and desulfurizing the circulating flue gas from the second waste heat boiler; pressurizing a portion of circulating flue gas after dedusting and desulfurizing and feeding pressurized circulating flue gas into the methanation unit, and selling the rest externally after cooled down as carbon dioxide products;
wherein in an initial stage, the glass furnace uses natural gas as the fuel and air as the combustion aid, and after flue gas is produced, the carbon-based enriched oxygen mixed by the flue gas, the oxygen produced by the air separation unit, and the oxygen produced by the water electrolysis hydrogen production unit gradually replaces the air and is used as the combustion aid, after a certain period of cyclic enrichment, the carbon dioxide concentration of the circulating flue gas reaches the equilibrium, and the methanation unit, the reforming unit, the first waste heat boiler, the second waste heat boiler, and the dedusting and desulfurization unit are put into operation, and the system enters into a normal operation state.

Further, in operation (1), the oxygen produced by the air separation unit has a purity of 99.6v% or higher, a normal temperature, and a pressure of 0.2-0.3 MPa, and the oxygen is fed into the mixer; the nitrogen produced by the air separation unit has a purity of 99.9v% or higher, a normal temperature, and a pressure of 0.2-0.3 MPa, the portion of the nitrogen is depressurized to 0.05 MPa and fed into the tin bath of the glass furnace as the protective gas, and the rest is sold externally; and
the hydrogen produced by the water electrolysis hydrogen production unit has a purity of 99.8v% or higher, a temperature of 90-100°C, and a pressure of 2.5-3.0 MPa, and the hydrogen is fed into the methanation unit; the oxygen produced by the water electrolysis hydrogen production unit has a purity of 99.0v% or higher, a temperature of 90-100°C, and a pressure of 2.5-3.0 MPa, and the oxygen is depressurized to 0.2-0.3 MPa and fed into the mixer.

Further, in operation (2), the oxygen from the air separation unit has a purity of 99.6v% or higher, a normal temperature, and a pressure of 0.2-0.3 MPa; the oxygen from the water electrolysis hydrogen production unit has a purity of 99.0v% or higher, a temperature of 90-100°C, and a pressure of 0.2-0.3 MPa; the circulating flue gas from the glass furnace has a carbon dioxide concentration of 95.0v% or higher, a temperature of 550-650°C, and a pressure of 0.15-0.25 MPa; the oxygen from the air separation unit, the oxygen from the water electrolysis hydrogen production unit, and the circulating flue gas from the glass furnace are each regulated in flow rate via flow regulating valves, the carbon-based enriched oxygen has an oxygen content of 21-30v%, a temperature of 460-480°C, and a pressure of 0.05-0.10 MPa, and the oxygen content is adjusted according to different requirements of the glass furnace.

Further, in operation (3), the hydrogen from the water electrolysis hydrogen production unit has a purity of 99.8v% or higher, a temperature of 90-100°C, and a pressure of 2.5-3.0 MPa, the circulating flue gas from the dedusting and desulfurization unit has a carbon dioxide concentration of 95.0v% or higher, a temperature of 250-300°C, and a pressure of 2.5-3.0 MPa, the hydrogen and the circulating flue gas are fed into the methanation unit at a volume ratio of H₂ and CO₂ of 4:1, under an action of a nickel-based methanation catalyst, the hydrogen and the carbon dioxide are catalyzed to generate methane, while releasing a large amount of heat, the methane has a purity of 94.0v% or higher on a dry basis, a temperature of 390-410°C, a pressure of 2.5-3.0 MPa, and the methane containing saturated water vapor is fed into the first waste heat boiler.

Further, in operation (4), the methane containing saturated water vapor from the methanation unit has a temperature of 390-410°C, the temperature is lowered to 240-260°C after the waste heat recovery by the first waste heat boiler and steam with a pressure of 1.5 MPa is by-produced for external sale; and the methane containing saturated water vapor after the waste heat recovery is depressurized to 0.5-1.0 MPa and then fed into the reforming unit.

Further, in operation (5), the methane containing saturated water vapor from the first waste heat boiler has a methane purity of 94.0v% or higher on a dry basis, a temperature of 240-260°C, and a pressure of 0.5-1.0 MPa, and the circulating flue gas from the glass furnace has a carbon dioxide concentration of 95.0v% or higher, a temperature of 1300-1400°C, and a pressure of 0.5-1.0 MPa; the circulating flue gas from the glass furnace is divided into two ways to enter the reforming unit, one way enters the heat storage grid on the one side of the reforming unit, providing heat for the heat storage grid on the one side of the reforming unit to provide heat for the raw gas of the reforming unit in the next round, after the heat exchange, the temperature of the circulating flue gas is reduced to 250-300°C, and the pressure of the circulating flue gas is reduced to 0.1-0.2 MPa and then fed into the dedusting and desulfurization unit; the other way and the methane containing saturated water vapor from the first waste heat boiler are fed into the heat storage grid on the other side of the reforming unit at a volume ratio of CH₄ and CO₂ of 2:1, the heat storage grid on the other side of the reforming unit is heated by the circulating flue gas of the previous round, the two heat storage grids work in rotation, and the raw gas fed into the reforming unit is heated to 900-1000°C by the heat storage grid on the other side of the reforming unit, under a pressure of 0.5-1.0 MPa and a nickel-based catalyst, the methane, the carbon dioxide, and the water vapor undergo the methane reforming reaction to produce the carbon monoxide and the hydrogen, with a volume ratio of CO and H₂ of 3:5, an outlet temperature of 700-800°C, and a pressure of 0.5-1.0 MPa, and the carbon monoxide and the hydrogen are fed into the glass furnace as the fuel.

Further, in operation (6), the carbon monoxide and the hydrogen from the reforming unit have a volume ratio of CO and H₂ of 3:5, a temperature of 700-800°C, and a pressure of 0.5-1.0 MPa, and the carbon-based enriched oxygen from the mixer has an oxygen content of 21-30v%, a temperature of 460-480°C, and a pressure of 0.05-0.10 MPa; the carbon-based enriched oxygen from the mixer first passes through the heat storage grid of the one side of the glass furnace, the heat storage grid is heated by the previous round of circulating flue gas, the two sides of the heat storage grids work in rotation, and the carbon-based enriched oxygen from the mixer is heated to 700-800°C and then fed into the glass furnace; the fuel and the combustion aid have a volume ratio of 1:2; in the glass furnace, under a combustion of the carbon-based enriched oxygen, the carbon monoxide and the hydrogen undergo a combustion reaction, providing heat for the production of the molten glass in the glass furnace; and
after the flue gas of the glass furnace is circularly enriched for 3-5 hours, and the carbon dioxide concentration reaches the equilibrium, with the carbon dioxide concentration of 95.0v% or higher, a temperature of 1300-1400°C, and a pressure of 0.01-0.05 MPa; the circulating flue gas is divided into two ways, one way passes through the heat storage grid on the other side of the glass furnace, providing heat for the heat storage grid on the other side of the glass furnace to provide heat for the carbon-based enriched oxygen from the mixer in the next round, after the heat exchange, the circulating flue gas of this way is cooled down to 550-650°C and pressurized to 0.15-0.25 MPa, and the circulating flue gas is fed into the mixer and the second waste heat boiler; the other way does not pass through the heat storage grid of the glass furnace, with a temperature of 1300-1400°C, is pressurized to 0.5-1.0 MPa, and fed into the reforming unit.

Further, in operation (7), the circulating flue gas from the glass furnace has a carbon dioxide concentration of 95.0v% or higher, a temperature of 550-650°C, and a pressure of 0.15-0.25MPa, after the waste heat recovery by the second waste heat boiler, the temperature is reduced to 250-300°C, and the pressure is 0.1-0.2 MPa, and the steam with a pressure of 1.5-4.0 MPa is by-produced for external sale; and the circulating flue gas after the waste heat recovery is fed into the dedusting and desulfurization unit.

Further, the dedusting and desulfurization unit includes a high-temperature dust collector and a dry desulfurization device; the high-temperature dust collector adopts cyclone separating and dust removing, and the dry desulfurization device adopts zinc oxide desulfurization.

In operation (8), the circulating flue gas first passes through the high-temperature dust collector of the dedusting and desulfurization unit to remove dust, and then passes through the dry desulfurization device of the dedusting and desulfurization unit to remove hydrogen sulfide; the circulating flue gas from the second waste heat boiler has a carbon dioxide concentration of 95.0v% or higher, a temperature of 250-300°C, and a pressure of 0.1-0.2 MPa, and the circulating flue gas after dust removal and desulphurization has a carbon dioxide concentration of 95.0v% or higher, a temperature of 250-300°C, and a pressure of 0.05-0.10 MPa, a portion of which is pressurized to 2.5-3.0 MPa and fed into the methanation unit, and the rest is cooled down and sold externally as the carbon dioxide products.

Further, in the initial stage, the glass furnace adopts the natural gas as the fuel and the air as the combustion aid, and after the flue gas is produced, the carbon-based enriched oxygen mixed by the flue gas, the oxygen produced by the air separation unit, and the oxygen produced by the water electrolysis hydrogen production unit gradually replaces the air and is used as the combustion aid, and after 3-5 hours of cyclic enrichment, the carbon dioxide concentration of the circulating flue gas reaches the equilibrium, with a carbon dioxide concentration of 95.0v% or higher, the methanation unit, the reforming unit, the first waste heat boiler, the second waste heat boiler, and the dedusting and desulfurization unit are put into operation, and the system enters into the normal operation state.

Beneficial effects of the zero-carbon glass furnace process provided by the present disclosure include:
1. The present disclosure adopts green electricity to drive the air separation unit to produce green oxygen and green nitrogen, drive the water electrolysis hydrogen production unit to produce green hydrogen and green oxygen; the flue gas of the glass furnace is circularly enriched into a high concentration of carbon dioxide (95.0v% or higher), which then undergoes methanation reactions with green hydrogen to produce methane, methane, carbon dioxide, and water vapor undergo a reforming reaction to produce carbon monoxide and hydrogen; the carbon monoxide and hydrogen are used as the fuel of the glass furnace, the green oxygen and carbon dioxide are mixed into the carbon-based enriched oxygen as the combustion aid of the glass furnace, carbon-based enriched oxygen combustion is carried out in the glass furnace, and the excess carbon dioxide is sold externally, thereby realizing a goal of green, energy-efficient, virtually NOx-free, and zero-carbon.
2. The present disclosure adopts the green electricity produced by the photovoltaic power generation unit to drive the air separation unit to produce green oxygen and green nitrogen, and adopts the green electricity produced by the photovoltaic power generation unit to drive the water electrolysis hydrogen production unit to produce green hydrogen and green oxygen, which is environmentally friendly.
3. In the present disclosure, the flue gas of the glass furnace is circularly enriched into a gas with a carbon dioxide concentration of 95.0v% or higher as the raw gas for methanation, the raw gas for reforming, and the raw gas for carbon-based enriched oxygen combustion of glass furnace, and the excess carbon dioxide is used as a product for sale, thereby truly realizing a zero-carbon glass furnace.
4. The present disclosure uses the green hydrogen produced by the water electrolysis hydrogen production unit and high-concentration carbon dioxide (95.0v% or higher) enriched by flue gas as the raw gas for methanation to produce methane, which utilizes waste carbon dioxide and improves the utilization rate of waste resources.
5. The present disclosure uses methane produced by the methanation unit, high concentration of carbon dioxide (95.0v% or higher) enriched by flue gas, and water vapor as the raw gas to produce carbon monoxide and hydrogen through the reforming reaction, which increases the calorific value of the fuel and reduces the fuel consumption.
6. The present disclosure adopts the high concentration of carbon dioxide (95.0v% or higher) enriched by the flue gas to replace air for carbon-based oxygen-enriched combustion, which can make the combustion process produce virtually NOx-free, and at the same time, carbon dioxide is a greenhouse gas, which improves a thermal efficiency of the glass furnace.
7. In the present disclosure, the flue gas is circularly enriched into a high concentration of carbon dioxide (95.0v% or higher), creating conditions for low-cost carbon dioxide capture and making the zero-carbon glass furnace possible.
8. The present disclosure combines methanation, reforming, and the glass furnace to make full use of the heat of the flue gas of the glass furnace for the heat exchange of the raw gas, improving a heat utilization rate.

### BRIEF DESCRIPTION OF THE DRAWINGS

The present disclosure is further described in terms of exemplary embodiments. These exemplary embodiments are described in detail with reference to the drawings, and the drawings are not to scale. These embodiments are non-limiting schematic embodiments, in which like reference numerals represent similar structures throughout the several views of the drawings, wherein:

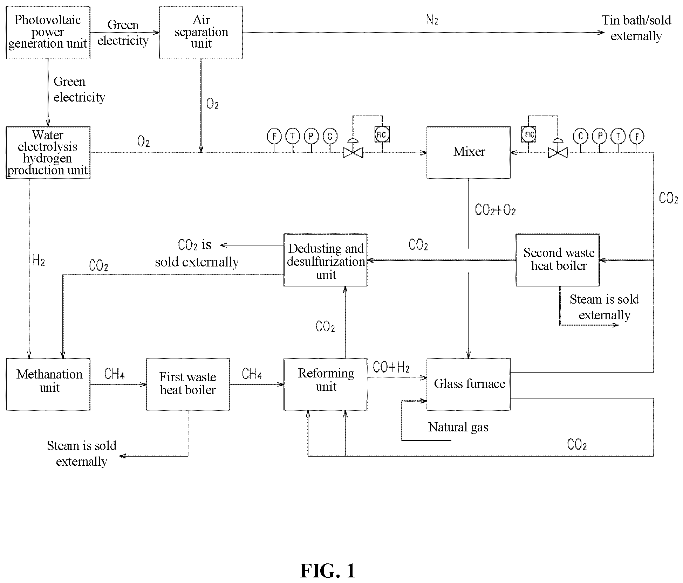

FIG. 1 is a schematic diagram illustrating a structure of a system required for a zero-carbon glass furnace process according to the present disclosure.

### DETAILED DESCRIPTION

In order to make the purpose, technical solutions and advantages of the embodiments of the present disclosure clearer, the technical solutions in the embodiments of the present disclosure will be clearly and completely described below in conjunction with the accompanying drawings in the embodiments of the present disclosure, and it is clear that the embodiments described is a part of the embodiments of the present disclosure, and not all of the embodiments. Accordingly, the following detailed description of the embodiments of the present disclosure provided in the accompanying drawings is not intended to limit the scope of the claimed protection, but only indicates selected embodiments of the present disclosure. Based on the embodiments in the present disclosure, all other embodiments obtained by a person skilled in the art without creative labor are within the protection scope of the present disclosure.

The present disclosure provides a zero-carbon glass furnace process, and a system required for the process is shown in FIG. 1, including a photovoltaic power generation unit, an air separation unit, a water electrolysis hydrogen production unit, a mixer, a methanation unit, a first waste heat boiler, a reforming unit, a glass furnace, a second waste heat boiler, and a dedusting and desulfurization unit.

The photovoltaic power generation unit is configured to produce green electricity to supply electricity to the air separation unit and the water electrolysis hydrogen production unit. The main principle of photovoltaic power generation is the photoelectric effect of semiconductors. When irradiated by electromagnetic waves with a frequency higher than a specific frequency (which is called a threshold frequency), electrons within certain substances absorb energy and escape to form an electric current, that is, photo-generated electricity. The photovoltaic power generation has the advantages of safety, reliability, no noise, no polluting emission, absolute cleanliness, and no harm.

The air separation unit is configured to produce oxygen and nitrogen using the green electricity supplied by the photovoltaic power generation unit as electrical energy. The air separation unit produces oxygen and nitrogen by following operations. The air is first compressed, cooled, and liquefied, and then gas-liquid contact on the distillation tower plate is conducted for mass and heat exchange using the difference in the boiling points of the oxygen and nitrogen components, the high-boiling-point oxygen component constantly condenses into liquid from the vapor, and low-boiling-point nitrogen component constantly transfers to the vapor, so that the nitrogen content in the rising vapor constantly increases, and the oxygen content in the downward-flowing liquid becomes increasingly high, thereby separating oxygen and nitrogen. The oxygen produced by the air separation unit has a purity of 99.6v% or higher, a normal temperature, and a pressure of 0.2-0.3 MPa, and the nitrogen produced by the air separation unit has a purity of 99.9v% or higher, a normal temperature, and a pressure of 0.2-0.3 MPa. Since the air separation unit adopts the green electricity produced by the photovoltaic power generation unit as the electrical energy, the produced oxygen is green oxygen, and the produced nitrogen is green nitrogen. The oxygen produced by the air separation unit is fed into the mixer, and mixed with the oxygen from the water electrolysis hydrogen production unit and circulating flue gas (a carbon dioxide concentration of the circulating flue gas is 95.0v% or higher) from the glass furnace to form carbon-based enriched oxygen for use in the glass furnace to aid in combustion. A portion of nitrogen produced by the air separation unit is depressurized to 0.05 MPa and then fed into the tin bath of the glass furnace as a protective gas, and the rest is sold externally.

The water electrolysis hydrogen production unit, powered by the green electricity supplied by the photovoltaic power generation unit, is configured to produce hydrogen and oxygen. Hydrogen production by water electrolysis is one of the convenient methods of producing hydrogen, in which direct current is passed through an electrolyzer filled with electrolyte, and water molecules react electrochemically on electrodes to decompose into hydrogen and oxygen. The hydrogen produced by the water electrolysis hydrogen production unit has a purity of 99.8v% or higher, a temperature of 90-100°C, and a pressure of 2.5-3.0 MPa, and the oxygen produced by the water electrolysis hydrogen production unit has a purity of 99.0v% or higher, a temperature of 90-100°C, and a pressure of 2.5-3.0 MPa. Since the water electrolysis hydrogen production unit uses the green electricity produced by the photovoltaic power generation unit as the electrical energy, the produced hydrogen is green hydrogen, and the produced oxygen is green oxygen. The hydrogen produced by the water electrolysis hydrogen production unit is fed into the methanation unit for the methanation reaction. In some embodiments, the oxygen produced by the water electrolysis hydrogen production unit is depressurized to 0.2-0.3 MPa and fed into the mixer, and is mixed with the oxygen from the air separation unit and the circulating flue gas (the carbon dioxide concentration of the circulating flue gas is 95.0v% or higher) from the glass furnace to form the carbon-based enriched oxygen as a combustion aid for use in the glass furnace to aid in combustion.

Hydrogen production by water electrolysis reaction equation is 2H₂O→2H₂+O₂.

The mixer is configured to mix oxygen from the air separation unit, oxygen from the water electrolysis hydrogen production unit, and the circulating flue gas from the glass furnace to form the carbon-based enriched oxygen as the combustion aid for use in the glass furnace to aid in combustion. The oxygen from the air separation unit has a purity of 99.6v% or higher, a normal temperature, and a pressure of 0.2-0.3 MPa. The oxygen from the water electrolysis hydrogen production unit has a purity of 99.0v% or higher, a temperature of 90-100°C, and a pressure of 0.2-0.3 MPa. The circulating flue gas from the glass furnace has a carbon dioxide concentration of 95.0v% or higher, a temperature of 550-650°C, and a pressure of 0.15-0.25 MPa. The oxygen from the air separation unit, the oxygen from the water electrolysis hydrogen production unit, and the circulating flue gas from the glass furnace are adjusted through corresponding flow regulating valve, respectively and mixed to form the carbon-based enriched oxygen with an oxygen content of 21-30v%, a temperature of 460-480°C, and a pressure of 0.05-0.10 MPa, and used as the combustion aid of the glass furnace. The oxygen content may be adjusted according to the different requirements of glass furnaces.

The methanation unit is configured to methanize hydrogen from the water electrolysis hydrogen production unit and circulating flue gas from the dedusting and desulfurization unit. The hydrogen from the water electrolysis hydrogen production unit has a purity of 99.8v% or higher, a temperature of 90-100°C, and a pressure of 2.5-3.0 MPa. The circulating flue gas from the dedusting and desulfurization unit has a carbon dioxide concentration of 95.0v% or higher, a temperature of 250-300°C, and a pressure of 2.5-3.0 MPa. The hydrogen and the carbon dioxide are fed into the methanation unit at a volume ratio of H₂ and CO₂ of 4:1, and under an action of a nickel-based methanation catalyst, the hydrogen and the carbon dioxide are catalyzed to generate methane containing saturated water vapor, while releasing a large amount of heat, the methane has a purity of 94.0v% or higher (on a dry basis), a temperature of 390-410°C, and a pressure of 2.5-3.0 MPa, and the methane (containing saturated water vapor) is fed into a first waste heat boiler.

Methanation reaction equation is 4H₂+CO₂→CH₄+2H₂O.

The first waste heat boiler is configured to recover waste heat of methane (containing saturated water vapor) from the methanation unit and by-produce steam. The methane (containing saturated water vapor) from the methanation unit has a temperature of 390-410°C, the temperature is lowered to 240-260°C after waste heat recovery from the first waste heat boiler and steam with a pressure of 1.5 MPa is by-produced for external sale. Since a suitable pressure for the reforming reaction is 0.5-1.0 MPa, the methane (containing saturated water vapor) after waste heat recovery is depressurized to 0.5-1.0 MPa and then fed into the reforming unit.

The reforming unit is configured to subject the methane (containing saturated water vapor) from the first waste heat boiler and a portion of the circulating flue gas from the glass furnace to a methane reforming reaction. The methane containing saturated water vapor from the first waste heat boiler has a methane purity of 94.0v% or higher (on a dry basis), a temperature of 240-260°C, and a pressure of 0.5-1.0 MPa. The circulating flue gas from the glass furnace has a carbon dioxide concentration of 95.0v% or higher, a temperature of 1300-1400°C, and a pressure of 0.5-1.0 MPa. The circulating flue gas from the glass furnace is divided into two ways to enter the reforming unit, one way enters the heat storage grid on the one side of the reforming unit, providing heat for the heat storage grid on the one side of the reforming unit to provide heat for the raw gas of the reforming unit in the next round, after heat exchange, the temperature of the circulating flue gas is reduced to 250-300°C, and the pressure of the circulating flue gas is reduced to 0.1-0.2 MPa and then fed into the dedusting and desulfurization unit; the other way and the methane (containing saturated water vapor) from the first waste heat boiler are fed into the heat storage grid on the other side of the reforming unit at a volume ratio of CH₄ and CO₂ of 2:1 (the heat storage grid on the other side of the reforming unit is heated by the circulating flue gas of the previous round, the two heat storage grids work in rotation), and the raw gas fed into the reforming unit is heated to 900-1000°C by the heat storage grid on the other side of the reforming unit, under a pressure of 0.5-1.0 MPa and the nickel-based catalyst, the methane, the carbon dioxide, and the water vapor undergo the methane reforming reaction in the reaction bed of the reforming unit to produce the carbon monoxide and the hydrogen, with a volume ratio of CO and H₂ of 3:5, an outlet temperature of 700-800°C, and a pressure of 0.5-1.0 MPa, and the carbon monoxide and the hydrogen are fed into the glass furnace as the fuel.

Reforming reaction equation is CH₄+H₂O→CO+3H₂ and CH₄+CO₂→2CO+2H₂.

The glass furnace is configured to produce the molten glass using carbon monoxide and hydrogen from the reforming unit as the fuel (natural gas is used as the fuel in an initial stage of production) and the carbon-based enriched oxygen from the mixer as the combustion aid. The carbon monoxide and the hydrogen from the reforming unit have a volume ratio of CO and H₂ of about 3:5, a temperature of 700-800°C, and a pressure of 0.5-1.0 MPa. The carbon-based enriched oxygen from the mixer has an oxygen content of 21-30v%, a temperature of 460-480°C, and a pressure of 0.05-0.10 MPa; the carbon-based enriched oxygen from the mixer first passes through the heat storage grid of the one side of the glass furnace (the heat storage grid is heated by the previous round of circulating flue gas, the two sides of the heat storage grids work in rotation), and the carbon-based enriched oxygen from the mixer is heated to 700-800°C and then fed into the glass furnace. The fuel (the carbon monoxide and the hydrogen from the reforming unit) and the combustion aid (the carbon-based enriched oxygen from the mixer) have a volume ratio of about 1:2. Under a combustion of the carbon-based enriched oxygen, the carbon monoxide and the hydrogen undergo a combustion reaction, providing heat for the production of the molten glass in the glass furnace.

Carbon-based enriched oxygen combustion reaction equation is 2CO+O₂+CO₂→3CO₂ and 2H₂+O₂+CO₂→2H₂O+CO₂.

After the flue gas of the glass furnace is circularly enriched 3-5 hours, the carbon dioxide concentration reaches the equilibrium, with the carbon dioxide concentration of 95.0v% or higher, a temperature of 1300-1400°C, and a pressure of 0.01-0.05 MPa. The circulating flue gas is divided into two ways, one way passes through the heat storage grid on the other side of the glass furnace, providing heat for the heat storage grid on the other side of the glass furnace to provide heat for the carbon-based enriched oxygen from the mixer in the next round, after heat exchange, the circulating flue gas of this way is cooled down to 550-650°C and pressurized to 0.15-0.25 MPa, and the circulating flue gas is fed into the mixer and the second waste heat boiler; the other way does not pass through the heat storage grid of the glass furnace, with a temperature of 1300-1400°C, is pressurized to 0.5-1.0 MPa, and fed into the reforming unit.

The second waste heat boiler is configured to recover waste heat of the circulating flue gas from the glass furnace and by-produce steam. The circulating flue gas from the glass furnace has a carbon dioxide concentration of 95.0v% or higher, a temperature of 550-650°C, and a pressure of 0.15-0.25MPa, after waste heat recovery by the second waste heat boiler, the temperature is reduced to 250-300°C, and the pressure is 0.1-0.2 MPa, and the steam with a pressure of 1.5-4.0 MPa is by-produced for external sale. The circulating flue gas after waste heat recovery is fed into the dedusting and desulfurization unit.

The dedusting and desulfurization unit is configured to dedust and desulfurize circulating flue gas from the second waste heat boiler. The dedusting and desulfurization unit includes a high-temperature dust collector and a dry desulfurization device. The high-temperature dust collector adopts cyclone separating and dust removing, and the dry desulfurization device adopts zinc oxide desulfurization. The circulating flue gas first passes through the high-temperature dust collector of the dedusting and desulfurization unit to remove dust, and then passes through the dry desulfurization device of the dedusting and desulfurization unit to remove hydrogen sulfide. The circulating flue gas from the second waste heat boiler has a carbon dioxide concentration of 95.0v% or higher, a temperature of 250-300°C, and a pressure of 0.1-0.2 MPa. The circulating flue gas after dust removal and desulphurization has a carbon dioxide concentration of 95.0v% or higher, a temperature of 250-300°C, and a pressure of 0.05-0.10 MPa, a portion of which is pressurized to 2.5-3.0 MPa and fed into the methanation unit, and the rest is cooled down and sold externally as the carbon dioxide products.

Dry desulfurization reaction equation is ZnO+H₂S→ZnS+H₂O.

The photovoltaic power generation unit is connected to the air separation unit and the water electrolysis hydrogen production unit, respectively; a nitrogen outlet of the air separation unit is connected to a tin bath of the glass furnace and to a nitrogen product storage tank, respectively, and a pressure reducing valve is provided on a connecting pipe between the nitrogen outlet of the air separation unit and the tin bath of the glass furnace; an oxygen outlet of the air separation unit and an oxygen outlet of the water electrolysis hydrogen production unit are both connected to the mixer, a pressure reducing valve is provided on a connecting pipe between the oxygen outlet of the water electrolysis hydrogen production unit and the mixer, a connecting pipe between the oxygen outlet of the air separation unit, the oxygen outlet of the water electrolysis hydrogen production unit, and the mixer is provided with a flow detector F, a temperature detector T, a pressure detector P, a gas (oxygen) purity detector C, and a flow regulating valve FIC; a hydrogen outlet of the water electrolysis hydrogen production unit is connected to the methanation unit; a carbon-based enriched oxygen outlet of the mixer is switchably connected to a heat storage grid A/B of the glass furnace and then connected to the glass furnace; a methane outlet of the methanation unit is connected to the first waste heat boiler; a methane outlet of the first waste heat boiler is switchably connected to a heat storage grid A/B of the reforming unit and then connected to the reforming unit, a pressure reducing valve is provided on a connecting pipe between the methane outlet of the first waste heat boiler and the heat storage grid A/B of the reforming unit, and a steam outlet of the first waste heat boiler is connected to a steam utilizer; a carbon monoxide and hydrogen outlet of the reforming unit is connected to the glass furnace; one of circulating flue gas outlets of the glass furnace is switchably connected to the heat storage grid B/A of the glass furnace and then connected to the second waste heat boiler and the mixer, one of the circulating flue gas outlets of the glass furnace is switchably connected to the heat storage grid A/B of the reforming unit and then connected to the reforming unit, and one of the circulating flue gas outlets of the glass furnace is switchably connected to a heat storage grid B/A of the reforming unit and then connected to the dedusting and desulfurization unit, respectively, a pressure booster is provided on a connecting pipe between the heat storage grid B/A of the glass furnace, the second waste heat boiler, and the mixer, a connecting pipe between the heat storage grid B/A of the glass furnace and the mixer is provided with a flow detector F, a temperature detector T, a pressure detector P, a gas (carbon dioxide) purity detector C, and a flow regulating valve FIC, a pressure booster is provided on the connecting pipe between the glass furnace, the heat storage grid A/B of the reforming unit, and the heat storage grid B/A of the reforming unit, and a pressure reducing valve is provided on a connecting pipe between the heat storage grid B/A of the reforming unit and the dedusting and desulfurization unit; a circulating flue gas outlet of the second waste heat boiler is connected to the dedusting and desulfurization unit; circulating flue gas outlets of the dedusting and desulfurization unit are connected to the methanation unit and a carbon dioxide product storage tank, respectively, and a pressure booster is provided on a connecting pipe between one of the circulating flue gas outlets of the dedusting and desulfurization unit and the methanation unit.

The process includes the following operations.
(1) The green electricity is produced by the photovoltaic power generation unit to supply the electricity for the air separation unit and the water electrolysis hydrogen production unit. The air separation unit produces oxygen and nitrogen with the green electricity provided by the photovoltaic power generation unit as the electrical energy, the oxygen has a purity of 99.6v% or higher, a normal temperature, and a pressure of 0.2-0.3 MPa, and the oxygen is fed into the mixer; and the nitrogen has a purity of 99.9v% or higher, a normal temperature, and a pressure of 0.2-0.3 MPa, the portion of the nitrogen is depressurized to 0.05 MPa and fed into the tin bath of the glass furnace as the protective gas, and the rest is sold externally. The water electrolysis hydrogen production unit produces oxygen and nitrogen with the green electricity provided by the photovoltaic power generation unit as the electrical energy, the hydrogen has a purity of 99.8v% or higher, a temperature of 90-100°C, and a pressure of 2.5-3.0 MPa, and the hydrogen is fed into the methanation unit; and the oxygen has a purity of 99.0v% or higher, a temperature of 90-100°C, and a pressure of 2.5-3.0 MPa, and the oxygen is depressurized to 0.2-0.3 MPa and fed into the mixer.
(2) The mixer mixes the oxygen from the air separation unit, the oxygen from the water electrolysis hydrogen production unit, and the circulating flue gas from the glass furnace into the carbon-based enriched oxygen as the combustion aid for the glass furnace. The oxygen from the air separation unit has a purity of 99.6v% or higher, a normal temperature, and a pressure of 0.2-0.3 MPa. The oxygen from the water electrolysis hydrogen production unit has a purity of 99.0v% or higher, a temperature of 90-100°C, and a pressure of 0.2-0.3 MPa. The circulating flue gas from the glass furnace has a carbon dioxide concentration of 95.0v% or higher, a temperature of 550-650°C, and a pressure of 0.15-0.25 MPa. The oxygen from the air separation unit, the oxygen from the water electrolysis hydrogen production unit, and the circulating flue gas from the glass furnace are each regulated in flow rate via flow regulating valves, the carbon-based enriched oxygen has an oxygen content of 21-30v%, a temperature of 460-480°C, and a pressure of 0.05-0.10 MPa, and the oxygen content is adjusted according to different requirements of the glass furnace.
(3) The hydrogen from the water electrolysis hydrogen production unit and the circulating flue gas from the dedusting and desulfurization unit are methanized by the methanation unit. The hydrogen from the water electrolysis hydrogen production unit has a purity of 99.8v% or higher, a temperature of 90-100°C, and a pressure of 2.5-3.0 MPa. The circulating flue gas from the dedusting and desulfurization unit has a carbon dioxide concentration of 95.0v% or higher, a temperature of 250-300°C, and a pressure of 2.5-3.0 MPa. The hydrogen and the circulating flue gas are fed into the methanation unit at a volume ratio of H₂ and CO₂ of 4:1, under an action of a nickel-based methanation catalyst, the hydrogen and the carbon dioxide are catalyzed to generate methane, while releasing a large amount of heat, the methane has a purity of 94.0v% or higher (on a dry basis), a temperature of 390-410°C, and a pressure of 2.5-3.0 MPa, and the methane (containing saturated water vapor) is fed into the first waste heat boiler.
(4) The waste heat of the methane (containing saturated water vapor) from the methanation unit is recovered and the steam is by-produced by the first waste heat boiler. The methane (containing saturated water vapor) from the methanation unit has a temperature of 390-410°C, the temperature is lowered to 240-260°C after waste heat recovery by the first waste heat boiler and steam with a pressure of 1.5 MPa is by-produced for external sale. The methane (containing saturated water vapor) after the waste heat recovery is depressurized to 0.5-1.0 MPa and feed into the reforming unit.
(5) The methane (containing saturated water vapor) from the first waste heat boiler and the portion of the circulating flue gas from the glass furnace are subjected to the methane reforming reaction by the reforming unit. The methane (containing saturated water vapor) from the first waste heat boiler has a methane purity of 94.0v% or higher (on a dry basis), a temperature of 240-260°C, and a pressure of 0.5-1.0 MPa. The circulating flue gas from the glass furnace has a carbon dioxide concentration of 95.0v% or higher, a temperature of 1300-1400°C, and a pressure of 0.5-1.0 MPa. The circulating flue gas from the glass furnace is divided into two ways to enter the reforming unit, one way enters the heat storage grid on the one side of the reforming unit, providing heat for the heat storage grid on the one side of the reforming unit to provide heat for the raw gas of the reforming unit in the next round, after heat exchange, the temperature of the circulating flue gas is reduced to 250-300°C, and the pressure of the circulating flue gas is reduced to 0.1-0.2 MPa and then fed into the dedusting and desulfurization unit; the other way and the methane (containing saturated water vapor) from the first waste heat boiler are fed to the heat storage grid on the other side of the reforming unit at a volume ratio of CH₄ and CO₂ of 2:1 (the heat storage grid on the other side of the reforming unit is heated by the circulating flue gas of the previous round, the two heat storage grids work in rotation), and the raw gas fed into the reforming unit is heated to 900-1000°C by the heat storage grid on the other side of the reforming unit, under a pressure of 0.5-1.0 MPa and a nickel-based catalyst, the methane, the carbon dioxide, and the water vapor undergo the methane reforming reaction in the reaction bed of the reforming unit to produce the carbon monoxide and the hydrogen, with a volume ratio of CO and H₂ of about 3:5, an outlet temperature of 700-800°C, and a pressure of 0.5-1.0 MPa, and the carbon monoxide and the hydrogen are fed into the glass furnace as the fuel.
(6) The molten glass is produced by the glass furnace using the carbon monoxide and the hydrogen from the reforming unit as the fuel (natural gas is used as the fuel during the initial stage of production) and the carbon-based enriched oxygen from the mixer as the combustion aid. The carbon monoxide and the hydrogen from the reforming unit have a volume ratio of CO and H₂ of about 3:5, a temperature of 700-800°C, and a pressure of 0.5-1.0 MPa. The carbon-based enriched oxygen from the mixer has an oxygen content of 21-30v%, a temperature of 460-480°C, and a pressure of 0.05-0.10 MPa; the carbon-based enriched oxygen from the mixer first passes through the heat storage grid of the one side of the glass furnace (the heat storage grid is heated by the previous round of circulating flue gas, the two sides of the heat storage grids work in rotation), and the carbon-based enriched oxygen from the mixer is heated to 700-800°C and then fed into the glass furnace. The fuel (the carbon monoxide and the hydrogen from the reforming unit) and the combustion aid (the carbon-based enriched oxygen from the mixer) have a volume ratio of about 1:2. In the glass furnace, the carbon monoxide and the hydrogen undergo a combustion reaction under the combustion support by the carbon-based enriched oxygen, which provides heat for the production of the molten glass in the glass furnace.

After the flue gas of the glass furnace is circularly enriched for 3-5 hours, the carbon dioxide concentration reaches the equilibrium, with the carbon dioxide concentration of 95.0v% or higher, a temperature of 1300-1400°C, and a pressure of 0.01-0.05 MPa. The circulating flue gas is divided into two ways, one way passes through the heat storage grid on the other side of the glass furnace, providing heat for the heat storage grid on the other side of the glass furnace to provide heat for the carbon-based enriched oxygen from the mixer in the next round, after heat exchange, the circulating flue gas of this way is cooled down to 550-650°C and pressurized to 0.15-0.25 MPa, and the circulating flue gas is fed into the mixer and the second waste heat boiler; the other way does not pass through the heat storage grid of the glass furnace, with a temperature of 1300-1400°C, is pressurized to 0.5-1.0 MPa, and fed into the reforming unit.

(7) The waste heat of the circulating flue gas from the glass furnace is recovered and the steam is by-produced by the second waste heat boiler. The circulating flue gas from the glass furnace has a carbon dioxide concentration of 95.0v% or higher, a temperature of 550-650°C, and a pressure of 0.15-0.25MPa, after waste heat recovery by the second waste heat boiler, the temperature is reduced to 250-300°C, and the pressure is 0.1-0.2 MPa, and the steam with a pressure of 1.5-4.0 MPa is by-produced for external sale. The circulating flue gas after waste heat recovery is fed into the dedusting and desulfurization unit.

(8) The circulating flue gas from the second waste heat boiler is dedusted and desulfurized by the dedusting and desulfurization unit. The circulating flue gas first passes through the high-temperature dust collector of the dedusting and desulfurization unit to remove dust, and then passes through the dry desulfurization device of the dedusting and desulfurization unit to remove hydrogen sulfide. The circulating flue gas from the second waste heat boiler has a carbon dioxide concentration of 95.0v% or higher, a temperature of 250-300°C, and a pressure of 0.1-0.2 MPa. The circulating flue gas after dust removal and desulphurization has a carbon dioxide concentration of 95.0v% or higher, a temperature of 250-300°C, and a pressure of 0.05-0.10 MPa, a portion of which is pressurized to 2.5-3.0 MPa and fed into the methanation unit, and the rest is cooled down and sold externally as the carbon dioxide products.

In the initial stage, the glass furnace adopts the natural gas as the fuel and the air as the combustion aid, and after the flue gas is produced, the carbon-based enriched oxygen mixed by the flue gas, the oxygen (produced by the air separation unit and the water electrolysis hydrogen production unit) gradually replaces the air and is used as the combustion aid, and after 3-5 hours of cyclic enrichment, the carbon dioxide concentration of the circulating flue gas reaches the equilibrium, with a carbon dioxide concentration of 95.0v% or higher, the methanation unit, the reforming unit, the first waste heat boiler, the second waste heat boiler, and the dedusting and desulfurization unit are put into operation, and the system enters into the normal operation state.

## Claims

1. A zero-carbon glass furnace process, wherein a system required for the process comprises a photovoltaic power generation unit, an air separation unit, a water electrolysis hydrogen production unit, a mixer, a methanation unit, a first waste heat boiler, a reforming unit, a glass furnace, a second waste heat boiler, and a dedusting and desulfurization unit; wherein
the photovoltaic power generation unit is configured to produce green electricity to supply electricity to the air separation unit and the water electrolysis hydrogen production unit;
the air separation unit is configured to produce oxygen and nitrogen;
the water electrolysis hydrogen production unit is configured to produce hydrogen and oxygen;
the mixer is configured to mix the oxygen from the air separation unit, the oxygen from the water electrolysis hydrogen production unit, and circulating flue gas from the glass furnace into carbon-based enriched oxygen;
the methanation unit is configured to methanize the hydrogen from the water electrolysis hydrogen production unit and circulating flue gas from the dedusting and desulfurization unit;
the first waste heat boiler is configured to recover waste heat of methane containing saturated water vapor from the methanation unit and by-produce steam;
the reforming unit is configured to subject the methane containing saturated water vapor from the first waste heat boiler and a portion of the circulating flue gas from the glass furnace to a methane reforming reaction;
the glass furnace is configured to produce molten glass using carbon monoxide and hydrogen from the reforming unit as fuel and the carbon-based enriched oxygen from the mixer as a combustion aid;
the second waste heat boiler is configured to recover waste heat of the circulating flue gas from the glass furnace and by-produce steam;
the dedusting and desulfurization unit is configured to dedust and desulfurize circulating flue gas from the second waste heat boiler;
the photovoltaic power generation unit is connected to the air separation unit and the water electrolysis hydrogen production unit, respectively; a nitrogen outlet of the air separation unit is connected to a tin bath of the glass furnace and to a nitrogen product storage tank, respectively, and a pressure reducing valve is provided on a connecting pipe between the nitrogen outlet of the air separation unit and the tin bath of the glass furnace; an oxygen outlet of the air separation unit and an oxygen outlet of the water electrolysis hydrogen production unit are both connected to the mixer, a pressure reducing valve is provided on a connecting pipe between the oxygen outlet of the water electrolysis hydrogen production unit and the mixer, a connecting pipe between the oxygen outlet of the air separation unit, the oxygen outlet of the water electrolysis hydrogen production unit, and the mixer is provided with a flow detector, a temperature detector, a pressure detector, an oxygen purity detector, and a flow regulating valve; a hydrogen outlet of the water electrolysis hydrogen production unit is connected to the methanation unit; a carbon-based enriched oxygen outlet of the mixer is switchably connected to a heat storage grid A/B of the glass furnace and then connected to the glass furnace; a methane outlet of the methanation unit is connected to the first waste heat boiler; a methane outlet of the first waste heat boiler is switchably connected to a heat storage grid A/B of the reforming unit and then connected to the reforming unit, a pressure reducing valve is provided on a connecting pipe between the methane outlet of the first waste heat boiler and the heat storage grid A/B of the reforming unit, and a steam outlet of the first waste heat boiler is connected to a steam utilizer; a carbon monoxide and hydrogen outlet of the reforming unit is connected to the glass furnace; one of circulating flue gas outlets of the glass furnace is switchably connected to the heat storage grid B/A of the glass furnace and then connected to the second waste heat boiler and the mixer, one of the circulating flue gas outlets of the glass furnace is switchably connected to the heat storage grid A/B of the reforming unit and then connected to the reforming unit, and one of the circulating flue gas outlets of the glass furnace is switchably connected to a heat storage grid B/A of the reforming unit and then connected to the dedusting and desulfurization unit, respectively, a pressure booster is provided on a connecting pipe between the heat storage grid B/A of the glass furnace, the second waste heat boiler, and the mixer, a connecting pipe between the heat storage grid B/A of the glass furnace and the mixer is provided with a flow detector, a temperature detector, a pressure detector, a carbon dioxide purity detector, and a flow regulating valve, a pressure booster is provided on the connecting pipe between the glass furnace, the heat storage grid A/B of the reforming unit, and the heat storage grid B/A of the reforming unit, and a pressure reducing valve is provided on a connecting pipe between the heat storage grid B/A of the reforming unit and the dedusting and desulfurization unit; a circulating flue gas outlet of the second waste heat boiler is connected to the dedusting and desulfurization unit; circulating flue gas outlets of the dedusting and desulfurization unit are connected to the methanation unit and a carbon dioxide product storage tank, respectively, and a pressure booster is provided on a connecting pipe between one of the circulating flue gas outlets of the dedusting and desulfurization unit and the methanation unit;
the process comprising operations of:
(1) the photovoltaic power generation unit producing the green electricity to supply the electricity for the air separation unit and the water electrolysis hydrogen production unit; the air separation unit producing the oxygen and the nitrogen with the green electricity supplied by the photovoltaic power generation unit as the electricity, wherein the oxygen is fed into the mixer, a portion of the nitrogen is depressurized and then fed into the tin bath of the glass furnace as protective gas, and the rest is sold outside; the water electrolysis hydrogen production unit producing the hydrogen and the oxygen with the green electricity supplied by the photovoltaic power generation unit as the electricity, wherein the hydrogen is fed into the methanation unit, and the oxygen is fed into the mixer after depressurization;
(2) the mixer mixing the oxygen from the air separation unit, the oxygen from the water electrolysis hydrogen production unit, and the circulating flue gas from the glass furnace into the carbon-based enriched oxygen, and using the carbon-based enriched oxygen as the combustion aid for the glass furnace;
(3) the methanation unit methanizing the hydrogen from the water electrolysis hydrogen production unit and the circulating flue gas from the dedusting and desulfurization unit to produce the methane containing saturated water vapor, and feeding the methane containing saturated water vapor into the first waste heat boiler;
(4) the first waste heat boiler recovering the waste heat of the methane containing saturated water vapor from the methanation unit and by-producing the steam; selling the steam externally, and feeding depressurized methane containing saturated water vapor after waste heat recovery into the reforming unit;
(5) the reforming unit subjecting the methane containing saturated water vapor from the first waste heat boiler and the portion of the circulating flue gas from the glass furnace to the methane reforming reaction; the circulating flue gas from the glass furnace entering into the reforming unit in two ways, one way entering into a heat storage grid of one side of the reforming unit, providing heat for the heat storage grid of the one side of the reforming unit to provide heat for raw gas of the reforming unit in a next round, and after heat exchange, the circulating flue gas being depressurized and fed into the dedusting and desulfurization unit; the other way entering into a heat storage grid on the other side of the reforming unit along with the methane containing saturated water vapor from the first waste heat boiler, the heat storage grid on the other side of the reforming unit being already heated by the circulating flue gas of a previous round, two sides of the heat storage grids working in rotation, and the raw gas fed into the reforming unit being heated by heat of the heat storage grid on the other side of the reforming unit, in a reaction bed of the reforming unit, under a certain pressure and a certain catalyst, methane, carbon dioxide, and water vapor undergoing the methane reforming reaction to produce the carbon monoxide and the hydrogen, and feeding the carbon monoxide and the hydrogen into the glass furnace as the fuel;
(6) the glass furnace producing the molten glass using the carbon monoxide and the hydrogen from the reforming unit as the fuel and the carbon-based enriched oxygen from the mixer as the combustion aid; the carbon-based enriched oxygen from the mixer first passing through a heat storage grid on one side of the glass furnace, the heat storage grid on the one side of the glass furnace being already heated by the previous round of the circulating flue gas, two sides of the heat storage grids working in rotation, and the carbon-based enriched oxygen from the mixer being heated to a certain temperature and then fed into the glass furnace;
after the flue gas of the glass furnace being circularly enriched for a certain period of time, a carbon dioxide concentration reaching an equilibrium; the circulating flue gas being divided into two ways, one way passing through a heat storage grid on the other side of the glass furnace, providing heat for the heat storage grid on the other side of the glass furnace to provide heat for the carbon-based enriched oxygen from the mixer in the next round, and after heat exchange, the circulating flue gas of this way being pressurized and fed into the mixer and the second waste heat boiler; the other way not passing through the heat storage grid of the glass furnace, and being pressurized and fed into the reforming unit;
(7) the second waste heat boiler recovering the waste heat of the circulating flue gas from the glass furnace and by-producing the steam; selling the steam externally, and feeding the circulating flue gas after waste heat recovery into the dedusting and desulfurization unit; and
(8) the dedusting and desulfurization unit dedusting and desulfurizing the circulating flue gas from the second waste heat boiler; pressurizing a portion of circulating flue gas after dedusting and desulfurizing and feeding pressurized circulating flue gas into the methanation unit, and selling the rest externally after cooled down as carbon dioxide products;
wherein in an initial stage, the glass furnace uses natural gas as the fuel and air as the combustion aid, and after flue gas is produced, the carbon-based enriched oxygen mixed by the flue gas, the oxygen produced by the air separation unit, and the oxygen produced by the water electrolysis hydrogen production unit gradually replaces the air and is used as the combustion aid, after a certain period of cyclic enrichment, the carbon dioxide concentration of the circulating flue gas reaches the equilibrium, and the methanation unit, the reforming unit, the first waste heat boiler, the second waste heat boiler, and the dedusting and desulfurization unit are put into operation, and the system enters into a normal operation state.

2. The process according to claim 1, wherein in operation (1), the oxygen produced by the air separation unit has a purity of 99.6v% or higher, a normal temperature, and a pressure of 0.2-0.3 MPa, and the oxygen is fed into the mixer; the nitrogen produced by the air separation unit has a purity of 99.9v% or higher, a normal temperature, and a pressure of 0.2-0.3 MPa, the portion of the nitrogen is depressurized to 0.05 MPa and fed into the tin bath of the glass furnace as the protective gas, and the rest is sold externally; and
the hydrogen produced by the water electrolysis hydrogen production unit has a purity of 99.8v% or higher, a temperature of 90-100°C, and a pressure of 2.5-3.0 MPa, and the hydrogen is fed into the methanation unit; the oxygen produced by the water electrolysis hydrogen production unit has a purity of 99.0v% or higher, a temperature of 90-100°C, and a pressure of 2.5-3.0 MPa, and the oxygen is depressurized to 0.2-0.3 MPa and fed into the mixer.

3. The process according to claim 1, wherein in operation (2), the oxygen from the air separation unit has a purity of 99.6v% or higher, a normal temperature, and a pressure of 0.2-0.3 MPa; the oxygen from the water electrolysis hydrogen production unit has a purity of 99.0v% or higher, a temperature of 90-100°C, and a pressure of 0.2-0.3 MPa; the circulating flue gas from the glass furnace has a carbon dioxide concentration of 95.0v% or higher, a temperature of 550-650°C, and a pressure of 0.15-0.25 MPa; the oxygen from the air separation unit, the oxygen from the water electrolysis hydrogen production unit, and the circulating flue gas from the glass furnace are each regulated in flow rate via flow regulating valves, the carbon-based enriched oxygen has an oxygen content of 21-30v%, a temperature of 460-480°C, and a pressure of 0.05-0.10 MPa, and the oxygen content is adjusted according to different requirements of the glass furnace.

4. The process according to claim 1, wherein in operation (3), the hydrogen from the water electrolysis hydrogen production unit has a purity of 99.8v% or higher, a temperature of 90-100°C, and a pressure of 2.5-3.0 MPa, the circulating flue gas from the dedusting and desulfurization unit has a carbon dioxide concentration of 95.0v% or higher, a temperature of 250-300°C, and a pressure of 2.5-3.0 MPa, the hydrogen and the circulating flue gas are fed into the methanation unit at a volume ratio of H₂ and CO₂ of 4:1, under an action of a nickel-based methanation catalyst, the hydrogen and the carbon dioxide are catalyzed to generate methane, while releasing a large amount of heat, the methane has a purity of 94.0v% or higher on a dry basis, a temperature of 390-410°C, a pressure of 2.5-3.0 MPa, and the methane containing saturated water vapor is fed into the first waste heat boiler.

5. The process according to claim 1, wherein in operation (4), the methane containing saturated water vapor from the methanation unit has a temperature of 390-410°C, the temperature is lowered to 240-260°C after the waste heat recovery by the first waste heat boiler and steam with a pressure of 1.5 MPa is by-produced for external sale; and the methane containing saturated water vapor after the waste heat recovery is depressurized to 0.5-1.0 MPa and then fed into the reforming unit.

6. The process according to claim 1, wherein in operation (5), the methane containing saturated water vapor from the first waste heat boiler has a methane purity of 94.0v% or higher on a dry basis, a temperature of 240-260°C, and a pressure of 0.5-1.0 MPa, and the circulating flue gas from the glass furnace has a carbon dioxide concentration of 95.0v% or higher, a temperature of 1300-1400°C, and a pressure of 0.5-1.0 MPa; the circulating flue gas from the glass furnace is divided into two ways to enter the reforming unit, one way enters the heat storage grid on the one side of the reforming unit, providing heat for the heat storage grid on the one side of the reforming unit to provide heat for the raw gas of the reforming unit in the next round, after the heat exchange, the temperature of the circulating flue gas is reduced to 250-300°C, and the pressure of the circulating flue gas is reduced to 0.1-0.2 MPa and then fed into the dedusting and desulfurization unit; the other way and the methane containing saturated water vapor from the first waste heat boiler are fed into the heat storage grid on the other side of the reforming unit at a volume ratio of CH₄ and CO₂ of 2:1, the heat storage grid on the other side of the reforming unit is heated by the circulating flue gas of the previous round, the two heat storage grids work in rotation, and the raw gas fed into the reforming unit is heated to 900-1000°C by the heat storage grid on the other side of the reforming unit, under a pressure of 0.5-1.0 MPa and a nickel-based catalyst, the methane, the carbon dioxide, and the water vapor undergo the methane reforming reaction to produce the carbon monoxide and the hydrogen, with a volume ratio of CO and H₂ of 3:5, an outlet temperature of 700-800°C, and a pressure of 0.5-1.0 MPa, and the carbon monoxide and the hydrogen are fed into the glass furnace as the fuel.

7. The process according to claim 1, wherein in operation (6), the carbon monoxide and the hydrogen from the reforming unit have a volume ratio of CO and H₂ of 3:5, a temperature of 700-800°C, and a pressure of 0.5-1.0 MPa, and the carbon-based enriched oxygen from the mixer has an oxygen content of 21-30v%, a temperature of 460-480°C, and a pressure of 0.05-0.10 MPa; the carbon-based enriched oxygen from the mixer first passes through the heat storage grid of the one side of the glass furnace, the heat storage grid is heated by the previous round of circulating flue gas, the two sides of the heat storage grids work in rotation, and the carbon-based enriched oxygen from the mixer is heated to 700-800°C and then fed into the glass furnace; the fuel and the combustion aid have a volume ratio of 1:2; in the glass furnace, under a combustion of the carbon-based enriched oxygen, the carbon monoxide and the hydrogen undergo a combustion reaction, providing heat for the production of the molten glass in the glass furnace; and
after the flue gas of the glass furnace is circularly enriched for 3-5 hours, the carbon dioxide concentration reaches the equilibrium, with the carbon dioxide concentration of 95.0v% or higher, a temperature of 1300-1400°C, and a pressure of 0.01-0.05 MPa; the circulating flue gas is divided into two ways, one way passes through the heat storage grid on the other side of the glass furnace, providing heat for the heat storage grid on the other side of the glass furnace to provide heat for the carbon-based enriched oxygen from the mixer in the next round, after the heat exchange, the circulating flue gas of this way is cooled down to 550-650°C and pressurized to 0.15-0.25 MPa, and the circulating flue gas is fed into the mixer and the second waste heat boiler; the other way does not pass through the heat storage grid of the glass furnace, with a temperature of 1300-1400°C, is pressurized to 0.5-1.0 MPa, and fed into the reforming unit.

8. The process according to claim 1, wherein in operation (7), the circulating flue gas from the glass furnace has a carbon dioxide concentration of 95.0v% or higher, a temperature of 550-650°C, and a pressure of 0.15-0.25MPa, after the waste heat recovery by the second waste heat boiler, the temperature is reduced to 250-300°C, and the pressure is 0.1-0.2 MPa, and the steam with a pressure of 1.5-4.0 MPa is by-produced for external sale; and the circulating flue gas after the waste heat recovery is fed into the dedusting and desulfurization unit.

9. The process according to claim 1, wherein the dedusting and desulfurization unit includes a high-temperature dust collector and a dry desulfurization device; the high-temperature dust collector adopts cyclone separating and dust removing, and the dry desulfurization device adopts zinc oxide desulfurization; and
in operation (8), the circulating flue gas first passes through the high-temperature dust collector of the dedusting and desulfurization unit to remove dust, and then passes through the dry desulfurization device of the dedusting and desulfurization unit to remove hydrogen sulfide; the circulating flue gas from the second waste heat boiler has a carbon dioxide concentration of 95.0v% or higher, a temperature of 250-300°C, and a pressure of 0.1-0.2 MPa, and the circulating flue gas after dust removal and desulphurization has a carbon dioxide concentration of 95.0v% or higher, a temperature of 250-300°C, and a pressure of 0.05-0.10 MPa, a portion of which is pressurized to 2.5-3.0 MPa and fed into the methanation unit, and the rest is cooled down and sold externally as the carbon dioxide products.

10. The process according to claim 1, wherein in the initial stage, the glass furnace adopts the natural gas as the fuel and the air as the combustion aid, and after the flue gas is produced, the carbon-based enriched oxygen mixed by the flue gas, the oxygen produced by the air separation unit, and the oxygen produced by the water electrolysis hydrogen production unit gradually replaces the air and is used as the combustion aid, and after 3-5 hours of cyclic enrichment, the carbon dioxide concentration of the circulating flue gas reaches the equilibrium, with a carbon dioxide concentration of 95.0v% or higher, the methanation unit, the reforming unit, the first waste heat boiler, the second waste heat boiler, and the dedusting and desulfurization unit are put into operation, and the system enters into the normal operation state.
